(19) Europäisches Patentamt
European Patent Office
Office européen des brevets

(11) EP 0 729 957 A1

(12) **EUROPEAN PATENT APPLICATION**
published in accordance with Art. 158(3) EPC

(43) Date of publication:
**04.09.1996 Bulletin 1996/36**

(21) Application number: **94931166.6**

(22) Date of filing: **26.10.1994**

(51) Int. Cl.[6]: **C07D 401/12**, C07D 471/04, A61K 31/44

(86) International application number:
**PCT/JP94/01800**

(87) International publication number:
**WO 95/11897 (04.05.1995 Gazette 1995/19)**

(84) Designated Contracting States:
**AT BE CH DE DK ES FR GB GR IE IT LI LU MC NL PT SE**

(30) Priority: **29.10.1993 JP 272494/93**

(71) Applicant: **YOSHITOMI PHARMACEUTICAL INDUSTRIES, LTD.**
**Osaka-shi Osaka 541 (JP)**

(72) Inventors:
- **KAWAKITA, Takeshi,**
**Yoshitomi Pharmaceut. Ind. Ltd.**
**Chikujo-gun, Fukuoka 871 (JP)**
- **SANO, Mitsuharu,**
**Yoshitomi Pharmaceut. Ind. Ltd.**
**Chikujo-gun, Fukuoka 871 (JP)**
- **YUTOKU, Yuko,**
**Yoshitomi Pharmaceut. Ind. Ltd.**
**Chikujo-gun, Fukuoka 871 (JP)**
- **IKEDA, Yoshifumi,**
**Yoshitomi Pharmaceut. Ind. Ltd.**
**Chikujo-gun, Fukuoka 871 (JP)**
- **HAGA, Keiichiro,**
**Yoshitomi Pharmaceut. Ind. Ltd.**
**Chikujo-gun, Fukuoka 871 (JP)**

(74) Representative: **von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

# (54) PYRIDINE COMPOUND AND MEDICINAL USE THEREOF

(57) A pyridine compound represented by general formula (I) or a medicinally acceptable salt thereof, and a medicinal use thereof. In said formula (I) $R^1$ represents hydrogen, halogen, alkyl, alkoxy, hydroxy, alkoxycarbonyl, carboxy or haloalkyl; $R^2$ and $R^3$ represent each independently hydrogen, halogen or alkyl; $R^4$ represents hydrogen, alkoxycarbonyl, hydroxyalkyl or acyloxyalkyl; $R^5$ represents hydrogen or alkyl; X represents oxygen, sulfur, SO or $SO_2$; Y and Z represent each independently CH or N; L represents ethylene or vinylene; m represents 0, 1 or 2; and n represents an integer of 1 to 1,000; provided that n represents 1 when L is vinylene and that n represents an integer of 4 to 1,000 when $R^4$ is hydrogen, X is oxygen and L is ethylene. The compound and medicinally acceptable salts thereof are useful as medicines because they have an antiulcer effect, the effect of protecting gastro-intestinal cells, the effect of preventing recrudescence and recurrence of ulcer, and an antibacterial effect on Helicobacter pylori.

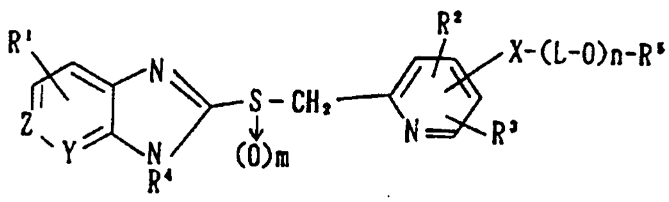

(I)



Printed by Rank Xerox (UK) Business Services
2.13.4/3.4

## Description

Technical Field

The present invention relates to novel pyridine compounds having antiulcer activity, gastrointestinal cytoprotective activity, ulcer recurrence or relapse-preventive activity, gastric acid secretion-suppressive activity, antibacterial activity against *Helicobacter pylori* and the like, pharmaceutically acceptable salts thereof and pharmaceutical use thereof.

Background Art

*Helicobacter pylori* (hereinafter sometimes referred to as *H. pylori*) is a Gram negative bacterium isolated from tunica mucosa ventriculi of a patient with active chronic gastritis (Warren, J.R. & Marshall, B.J. Lancet i: 1273-1275, 1983). *H. pylori* is a 0.5 µm wide, 3-5 µm long Gram negative spirally curved rod having several polar flagella and flagella sheaths at one or both ends of the cell. *H. pylori* grows under the microaerophilic environment, cannot grow under the aerobic conditions and grows poorly under the anaerobic conditions. It grows at 37°C and scarcely grows at lower than 25°C and higher than 42°C. The bacterium is notably characterized in that it evidently produces urease (Mobley, H.L. et al., Clin. Microbiol., *26*, 831-836, 1988).

*H. pylori* is present in the mucous layer in the tunica mucosa ventriculi epithelium of humans and swims in the viscous mucous layer using peculiar flagella that the bacterium has. *H. pylori* specifically resides in the cortical layer of the epithelium cells and the crevice therein, which provide the most comfortable living environment where gastric acidity is neutral, and lives by utilizing hemin, urea and so on (Hazell, S.L., Adrian, L., J. Infect. Dis., *153*, 658-663, 1986).

As to the mechanism of the development of diseases caused by *H. pylori*, mucous membrane disorder concept (Hazell, S.L., Adrian, L., J. Infect. Dis., *153*, 658-663, 1986), leaking roof concept (Goodwin C.S., Lancet ii, 1467-1469, 1988) and gastrin link concept (Levi, S. et al., Lancet i, 1167-1168, 1989) have been proposed.

The mucous membrane disorder caused by *H. pylori* is mostly ascribed to a strong urease activity (Hazell, S.L., Adrian, L., J. Infect. Dis., *153*, 658-663, 1986). The urea present in the gastric juice is decomposed by the urease of *H. pylori* to produce a large amount of ammonium and carbon dioxide. The ammonium concentration in the gastric juice is significantly high in the group that tested positive to *H. pylori*, and histological epitheliocyte disorder and increased ulcer coefficient have also been reported from a test where ammonium was orally administered to rats (Murakami, M. et al., Clin. Gastroenterol., *12* (Suppl. 1), S104-109, 1990). Also, there is a report on reduced amounts of PAS (periodic acid schiff)-positive mucosal juice in the tunica mucosa ventriculi of the patients who tested positive to *H. pylori*, suggesting possible degradation in the tunica mucosa ventriculi-protective activity due to the decomposition of mucosal juice by the protease of *H. pylori* (Nakajima, M. et al., Drug Investigation, *2* (Suppl. 1), *60*, 1990). It has been further reported that leukotriene B4 activity is high in the tunica mucosa ventriculi where prominent *H. pylori*-positive leukocyte infiltration is observed (Uchida, T. et al., Therapeutic Research, *12*, 85-90, 1991) and that the mucous membrane disorder is caused by the action of phospholipase A2 on bile acid which flows reversely into the stomach. In 1988, Leunk et al. reported the presence of a cytovacuolating toxin in the supernatant of *H. pylori* culture. The toxin was isolated and purified by Cover et al (Cover, T.L. & Blaser, M.J., J. Biol. Chem. *267*, 10570-10575, 1992). There are reports on the toxin that it delays cure of ulcer by hindering the rotation of cells near the ulcer (Chang, K. et al., Gastroenterology, *104* (Suppl.), A52, 1993) and that it acts synergistically with ammonium on cytovacuolation (Sommi, P. et al., Gastroenterology, *104* (Suppl.), A196, 1993). In recent years, disorder of tunica mucosa ventriculi, which is caused by acetoaldehyde produced in the stomach by the alcohol dehydrogenase activity possessed by *H. pylori* (Salmela, K.S. et al., Gastroenterology, *105*, 325-330, 1990), monochloramine produced in the presence of ammonium (Saida, H. et al., *Nihon Shokakibyo Gakkai Zasshi 90*, 1949, 1993) and interleukin 8 (Crowe, S.E. et al., Gastroenterology, *104*, A687, 1993) have been drawing attention.

With regard to the relationship between peptic ulcer and *H. pylori*, the involvement of *H. pylori* has been strongly suggested in view of the very high segregation frequency of *H. pylori*, particularly in duodenal ulcer, and infection with *H. pylori* is considered to be responsible for a prolonged cure and recurrence of peptic ulcer (see, for example, Raws EAJ, et al., Gastroenterology, *94*, 33-40, 1988).

With regard to the relationship between gastritis and *H. pylori*, the causality has been suggested based on oral infection tests in human (Morris, A. & Nicholoson, G. Am., J. Gastroenterology, *82*, 192-199, 1987, Marshall, B.J. et al., Med. J. Aust., *142*, 436-439, 1985). The segregation frequency of *H. pylori* from gastritis is high and the correlation has been confirmed between the cell number of *H. pylori*, and the increase in neutrophilic leukocytes (which is an index of activeness of gastritis) and the degree of clinical symptoms with infiltration of lymphocytes. Moreover, the development of gastritis in vestibulum of stomach with flare and erosion has been endoscopically observed in animal tests using Japanese monkeys (Shuto, R. et al., Infect. Immun., *61*, 933-939, 1993).

The number of reports on the onset of stomach tumor, which begins with persistent infection of *H. pylori* via atrophic gastritis and intestinal metaplasia, is recently on the rise. In 1991, Parsonnet et al. determined the IgG antibody titer of anti-*H. pylori* using a serum preserved for about 25 years and found a strong correlation between stomach tumor

and patients having the antibody. It has been also found, by epidemiological studies, that morbidity of and death from stomach tumor are high in the region where *H. pylori* antibody positive ratio is high (Parsonnet, J. et al., N. Engl. J. Med., *325*, 1127-1131, 1991).

The treatment of stomach ulcer and duodenal ulcer has made a remarkable progress over the recent years. However, the problems remain that they are susceptible to recurrence when drugs therefor are reduced or withdrawn, and that an intractable ulcer having resistance to drugs may be developed. With the prevailing indication of the involvement of *H. pylori* in stomach and duodenal ulcers, eradication of *H. pylori* by the administration of drugs having antibacterial activity against *H. pylori* has become the target of investigation (Chiba, N. et al., Am. J. Gastroenterology, *87*, 1716-1727, 1992).

The antibacterial activity against *H. pylori* is particularly prominent in amoxicillin and clarithromycin from among the antibacterial drugs. While the antibacterial activity can be also found in such antiprotozoals as metronidazole and tinidazole, resistance to these drugs is known to be acquired at a rather early stage. Of the antiulcer drugs, proton pump inhibitors such as omeprazole show weak antibacterial activity, and H2 receptor antagonists such as cimetidine, ranitidine and famotidine fail to show such activity. The drugs having an antibacterial activity exhibit only weak clinical effects by single administration. Therefore, administration of plural antibacterial drugs, dual therapy concurrently using a bismuth preparation and an antibacterial drug, or triple therapy using a bismuth preparation and two antibacterial drugs has been tried. Yet, these therapeutic methods rather frequently cause side-effects such as diarrhea and abdominal distension.

There are many reports supporting the effectiveness of the eradication of *H. pylori* and they agree on a conclusion that the eradication resulted in cure of intractable ulcer and suppression of ulcer recurrence.

Raws et al. achieved 88% eradication by the concurrent use of three drugs of bismuth subcitrate, amoxicillin and metronidazole and 6% recurrence ratio one year thereafter (Raws EAJ, et al., Gastroenterology, *94*, 33-40, 1988). Moreover, Graham et al. reportedly used three drugs of bismuth subcitrate, tetracycline and metronidazole along with the treatment using ranitidine, and achieved eradication in 89% of the ulcer cases and recurrence ratio after one year of 12% in duodenal ulcer and 13% in stomach ulcer, which ratios being remarkably lower than the recurrence ratios of 95% and 74% after the treatment solely with ranitidine (Graham, D.Y. et al., *Shokaki Naishikyo*, Vol. 4, 429-440, 1992). A therapy by the concurrent use of a proton pump inhibitor and an antibacterial drug has been tried in recent years and ulcer recurrence-preventive effect to the same degree as that achieved in the concurrent use of three antibacterial drugs was reportedly obtained (Fujioka, T. et al., *Nihon Rinsyo*, Vol. 51, 3255-3260, 1993).

Some report says that administration of an antibacterial drug for eradicating *H. pylori* to a patient with intractable ulcer, who failed to cure with H2 receptor antagonists, resulted in cure of the ulcer, thus suggesting a potential use of the administration as a treatment method as well as for the prevention of recurrence. As stated in the foregoing, the usefulness of eradicating *H. pylori* is evident, though many problems remain to be solved before complete eradication is achieved. For example, long-term administration of bismuth preparation, which is useful for the eradication of *H. pylori*, is not possible, due to diarrhea, nausea and side effects on central nervous system that it causes; bismuth subcitrate is not approved as an antiulcer drug in Japan; side-effects caused by the multiple drug therapy are frequently found; antibacterial drugs used for the multiple drug therapy amounts high; usable time periods of the antibacterial drugs have not been established yet; and re-infection after the eradication of *H. pylori* is found. In view of the problems as described, an antiulcer drug is desired which has a strong selective antibacterial action on *H. pylori* and less side-effects. It is needless to say that a drug having an antibacterial action only against *H. pylori*, and gastric acid secretion suppressive action as well, is desirable from the aspect of ulcer curing.

Japanese Patent Unexamined Publication Nos. 209809/1990, 38523/1991, 48680/1991, 52887/1991, 52812/1991 and International Publication No. WO92/12976 disclose compounds having antibacteria] activity against *Helicobacter pylori*. Similar compounds have been disclosed in International Publication No. WO93/24480 and Japanese Patent Unexamined Publication No. 100449/1994 which were published after the priority date of the present invention; however, a compound having more excellent activities than these compounds is demanded.

## Disclosure of the Invention

With the aim of solving the above-mentioned problems, the present inventors have conducted various studies and found a compound having not only selective and superior antibacterial activity against *Helicobacter pylori*, but also antiulcer activity, gastrointestinal cytoprotective activity, ulcer recurrence or relapse-preventive activity and gastric acid secretion-suppressive activity, which resulted in the completion of the invention.

Accordingly, the present invention relates to pyridine compounds of the formula (I)

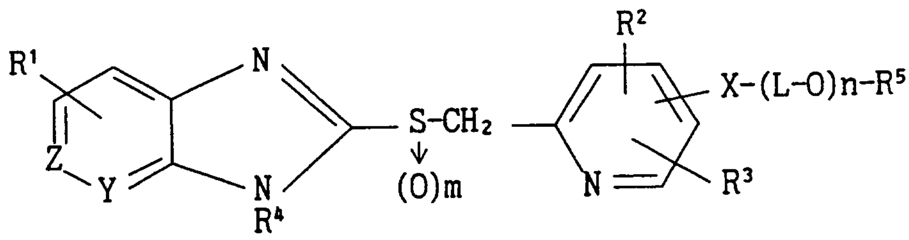

(I)

wherein

| | |
|---|---|
| $R^1$ | is a hydrogen, a halogen, an alkyl, an alkoxy, a hydroxyl, an alkoxycarbonyl, a carboxyl or a haloalkyl; |
| $R^2$ and $R^3$ | are the same or different and each is a hydrogen, a halogen or an alkyl; |
| $R^4$ | is a hydrogen, an alkoxycarbonyl, a hydroxyalkyl or an acyloxyalkyl; |
| $R^5$ | is a hydrogen or an alkyl; |
| X | is an oxygen atom, a sulfur atom, SO or $SO_2$; |
| Y and Z | are the same or different and each is CH or N; |
| L | is ethylene or vinylene; |
| m | is 0, 1 or 2; and |
| n | is an integer of from 1 to 1,000, |

provided that when L is vinylene, n is 1, and when $R^4$ is a hydrogen, X is an oxygen atom and L is ethylene, n is an integer of from 4 to 1,000 [hereinafter the compound is also referred to as compound (I)], and pharmaceutically acceptable salts thereof.

The present invention also relates to the above-mentioned pyridine compounds wherein the formula (I) is the formula (Ia)

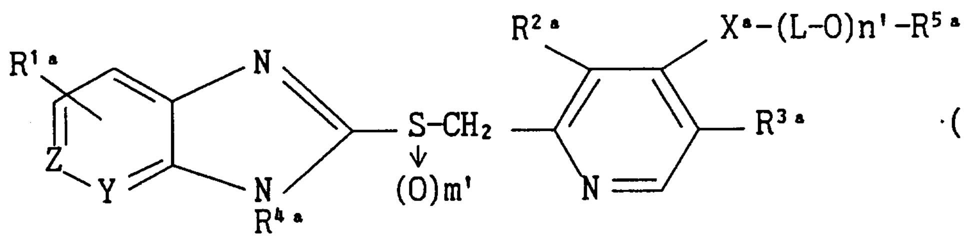

·(Ia)

wherein

| | |
|---|---|
| $R^{1a}$ | is a hydrogen, a halogen, an alkyl having 1 to 5 carbon atoms, an alkoxy having 1 to 5 carbon atoms or a hydroxyl; |
| $R^{2a}$ and $R^{3a}$ | are the same or different and each is a hydrogen or an alkyl having 1 to 5 carbon atoms; |
| $R^{4a}$ | is a hydrogen; |
| $R^{5a}$ | is a hydrogen or an alkyl having 1 to 8 carbon atoms; |
| $X^a$ | is an oxygen atom or a sulfur atom; |
| Y and Z | are the same or different and each is CH or N; |
| L | is ethylene or vinylene; |
| m' | is 0 or 1; and |
| n' | is an integer of from 1 to 100, |

provided that when L is vinylene, n' is 1, and when $X^a$ is an oxygen atom and L is ethylene, n' is an integer of from 4 to 100, and pharmaceutically acceptable salts thereof, and the above-mentioned pyridine compounds wherein the formula (I) is the formula (Ib)

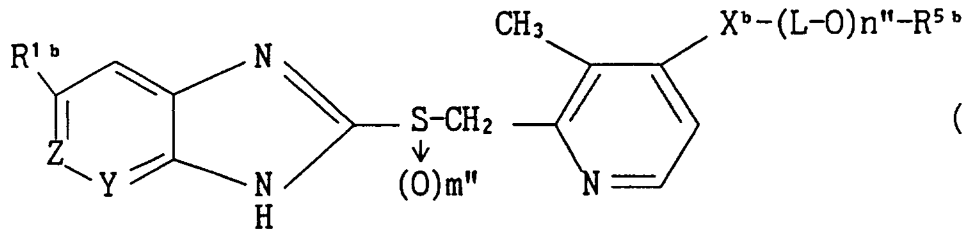

(Ib)

wherein

| | |
|---|---|
| $R^{1b}$ | is a hydrogen, a halogen, an alkoxy having 1 to 3 carbon atoms or hydroxyl; |
| $R^{5b}$ | is an alkyl having 1 to 5 carbon atoms; |
| $X^b$ | is an oxygen atom or a sulfur atom; |
| Y and Z | are the same or different and each is CH or N; |
| L | is ethylene or vinylene; |
| m" | is 0 or 1; and |
| n" | is an integer of from 1 to 20, |

provided that when L is vinylene, n" is 1, and when $X^b$ is an oxygen atom and L is ethylene, n" is an integer of from 4 to 20, and pharmaceutically acceptable salts thereof, in particular, the above-mentioned pyridine compounds wherein $X^b$ in the formula (Ib) is a sulfur atom and pharmaceutically acceptable salts thereof.

The present invention further relates to pharmaceutical compositions comprising the above-mentioned pyridine compound or a pharmaceutically acceptable salt thereof, and pharmaceutical additives; in particular, the present invention relates to agents for the prevention and treatment of various diseases caused by the bacteria belonging to the genus *Helicobacter* and agents for the prevention and treatment of gastrointestinal diseases.

Each symbol used in the present Specification is explained in the following.

With regard to $R^1$, halogen is chlorine, fluorine, bromine or iodine, alkyl is linear or branched alkyl having 1 to 20 carbon atoms, such as methyl, ethyl, propyl, isopropyl, butyl, isobutyl, tert-butyl, pentyl, hexyl, octyl, decyl, dodecyl, octadecyl and icosyl, with preference given to all having 1 to 5 carbon atoms.

Alkoxy is a linear or branched alkoxy having 1 to 20 carbon atoms, such as methoxy, ethoxy, propoxy, isopropoxy, butoxy, isobutoxy, tert-butoxy, pentyloxy, hexyloxy, octyloxy, decyloxy, dodecyloxy, octadecyloxy and icosyloxy, with preference given to alkoxy having 1 to 5 carbon atoms and particular preference given to alkoxy having 1 to 3 carbon atoms.

Alkoxycarbonyl is a linear or branched alkoxycarbonyl having 2 to 20 carbon atoms, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, octyloxycarbonyl, decyloxycarbonyl, dodecyloxycarbonyl, octadecyloxycarbonyl and icosyloxycarbonyl.

Haloalkyl is a linear or branched haloalkyl having 1 to 4 carbon atoms, such as trifluoromethyl, 2,2,2-trifluoroethyl, 2,3,3-trifluoropropyl, 1,1,2,2-tetrafluoroethyl and 2,2,3,3-tetrafluoropropyl.

With regard to $R^2$ and $R^3$, halogen is as exemplified above, and alkyl is as exemplified above, with preference given to alkyl having 1 to 5 carbon atoms. Particularly preferred is methyl.

With regard to $R^4$, alkoxycarbonyl is as exemplified above.

Hydroxyalkyl is a linear or branched hydroxyalkyl having 1 to 4 carbon atoms, such as hydroxymethyl, 1-hydroxyethyl, 2-hydroxyethyl, 3-hydroxypropyl, 2,3-dihydroxypropyl and 4-hydroxybutyl.

Acyloxyalkyl is that wherein the acyl moiety has 2 to 7 carbon atoms and the alkyl moiety has 1 to 4 carbon atoms. Examples thereof include acetyloxymethyl, propionyloxymethyl, 2-acetyloxyethyl, 3-acetyloxypropyl, 4-acetyloxybutyl, benzoyloxymethyl, 2-benzoyloxyethyl, and benzoyloxymethyl and 2-benzoyloxyethyl substituted by 1 to 3 substituents selected from halogen, alkyl, alkoxy, haloalkyl, hydroxyl, nitro and amino on a benzene ring. Halogen, alkyl, alkoxy and haloalkyl as substituents are as exemplified above.

Alkyl at $R^5$ is as exemplified above. Preferred are those having 1 to 8 carbon atoms and particularly preferred are those having 1 to 5 carbon atoms.

Halogen at $R^{1a}$ and $R^{1b}$ is as exemplified above. Alkyl at $R^{1a}$, $R^{2a}$, $R^{3a}$, $R^{5a}$ and $R^{5b}$ is exemplified by those mentioned above which have the corresponding carbon numbers. Alkoxy at $R^{1a}$ and $R^{1b}$ is exemplified by those mentioned above which have the corresponding carbon numbers.

Each group of formula (I) is preferably as follows:

$R^1$ is preferably a hydrogen, a halogen, an alkyl having 1 to 5 carbon atoms, an alkoxy having 1 to 5 carbon atoms or a hydroxyl, and is particularly preferably a hydrogen, a halogen, an alkyl having 1 to 3 carbon atoms or a hydroxyl;
$R^2$ is preferably a hydrogen or an alkyl having 1 to 5 carbon atoms, and is particularly preferably methyl;
$R^3$ is preferably a hydrogen or an alkyl having 1 to 5 carbon atoms, and is particularly preferably a hydrogen;
$R^4$ is preferably a hydrogen,
$R^5$ is preferably a hydrogen or an alkyl having 1 to 8 carbon atoms, and is particularly preferably an alkyl having 1 to 5 carbon atoms,
X is preferably an oxygen atom or a sulfur atom, and is particularly preferably a sulfur atom;
m is preferably 0 or 1; and
n is preferably an integer of from 1 to 100, and is particularly an integer of from 1 to 20; the substituent $R^1$ is preferably present at the 5-position when Y and Z are CH, and when Y or Z is N, the substituent is preferably absent, namely, $R^1$ is a hydrogen; and
the substituents $R^2$, $R^3$ and X-(L-O)n-$R^5$ are preferably at the 3-position for $R^2$, the 5-position for $R^3$ and the 4-position for X-(L-O)n-$R^5$.

The preferable compounds of the formula (I) are:

2-((3-methyl-4-(2-(2-methoxyethoxy)ethylthio)-2-pyridyl)methylthio)-1H-benzimidazole,
2-((3-methyl-4-(2-methoxypolyethoxy)-2-pyridyl)methylthio)-1H-benzimidazole derived from polyethylene glycol monomethyl ether having an average molecular weight of 350,
2-((3-methyl-4-(2-(2-(2-methoxyethoxy)ethoxy)ethylthio)-2-pyridyl)methylthio)-1H-benzimidazole,
2-((3-methyl-4-(2-(2-methoxypolyethoxy)ethylthio)-2-pyridyl)methylthio)-1H-benzimidazole derived from polyethylene glycol monomethyl ether having an average molecular weight of 350,
2-((3-methyl-4-(2-(2-methoxypolyethoxy)ethylthio)-2-pyridyl)methylthio)-1H-benzimidazole derived from polyethylene glycol monomethyl ether having an average molecular weight of 550,
2-((3-methyl-4-(2-(2-methoxypolyethoxy)ethylthio)-2-pyridyl)methylsulfinyl)-1H-benzimidazole derived from polyethylene glycol monomethyl ether having an average molecular weight of 350,
2-((3-methyl-4-(2-methoxypolyethoxy)-2-pyridyl)methylsulfinyl)-1H-benzimidazole derived from polyethylene glycol monomethyl ether having an average molecular weight of 350,
trans-2-((3-methyl-4-(2-ethoxyvinylthio)-2-pyridyl)methylthio)-1H-benzimidazole and
2-((3-methyl-4-(2-(2-methoxypolyethoxy)ethylthio)-2-pyridyl)methylthio)-3H-imidazo[4,5-b]pyridine derived from polyethylene glycol monomethyl ether having an average molecular weight of 350.

Pharmaceutically acceptable salts thereof are also preferable. Particularly preferable compounds are those of the formula (I) wherein X is a sulfur atom.

While the compound of the formula (I) of the present invention comprises various isomers, the present invention encompasses one of these isomers or a mixture of these isomers.

The pharmaceutically acceptable salt of the compound of the formula (I) includes acid addition salts such as hydrochloride, hydrobromide, hydroiodide, sulfate, phosphate, acetate, citrate, maleate, fumarate, malonate, malate, tartrate, succinate and methanesulfonate; alkali metal salts such as sodium salt and potassium salt; and alkaline earth metal salts such as calcium salt and magnesium salt.

The compound (I) of the present invention may exist as hydrates (e.g. semi-hydrate, monohydrate and sesquihydrate) or solvates, which are also encompassed in the present invention.

The compounds of the formula (I) can be produced by the following methods.

<u>Method 1:</u> A compound of the formula (II)

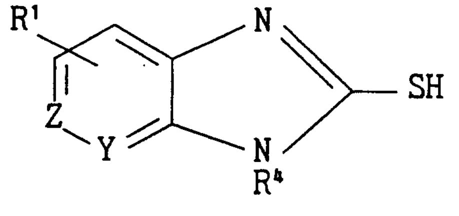

(II)

wherein each symbol is as defined above, is reacted with a compound of the formula (III)

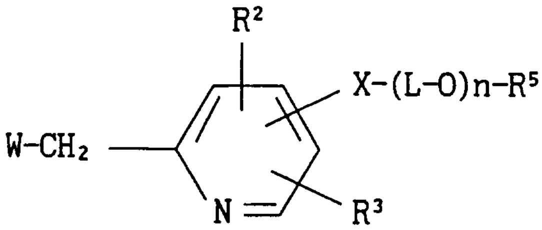

(III)

wherein W is a reactive atom or group such as halogen (as defined above) or sulfonyloxy group (e.g. methanesulfonyloxy, benzenesulfonyloxy and p-toluenesulfonyloxy) and other symbols are as defined above, or an acid addition salt thereof to give a compound of the formula (I-1)

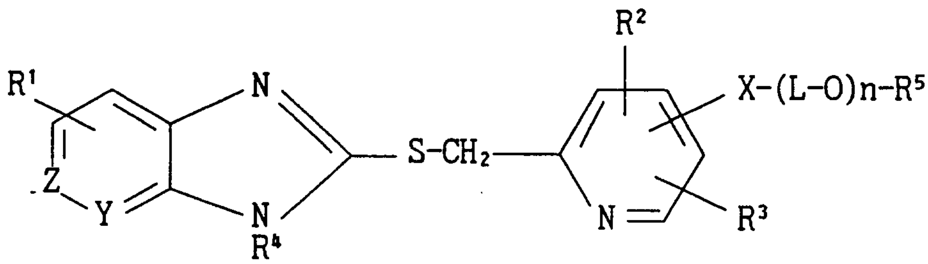

(I-1)

wherein each symbol is as defined above.

A compound of the formula (I) wherein m is 1 or 2 can be obtained by subjecting a compound of the formula (I-1) wherein m is 0 to oxidation.

The reaction between a compound (II) and a compound (III) or an acid addition salt thereof generally proceeds in a solvent inert to the reaction, such as water, methanol, ethanol, dimethylformamide and a mixed solvent thereof, preferably aqueous ethanol, in the presence of a base such as sodium hydroxide, potassium hydroxide, sodium hydride, potassium hydride, sodium methoxide, sodium carbonate, potassium carbonate, metallic sodium, triethylamine and pyridine, at a temperature of from about 0°C to the boiling point of the solvent used, preferably from 20°C to 80°C for about 10 minutes to 24 hours, preferably 30 minutes to 7 hours. Examples of the acid addition salt of the compound of the formula (III) include hydrochloride and hydrobromide.

Examples of the oxidizing agent to be used for the oxidation include meta-chloroperbenzoic acid, peracetic acid, trifluoroperacetic acid, permaleic acid, potassium hyperoxide, sodium bromite, sodium hypobromite and hydrogen peroxide.

Said oxidation reaction is generally carried out in a solvent inert to the reaction such as water, dichloromethane, chloroform, tetrahydrofuran, dioxane, dimethylformamide and a mixed solvent thereof, in the presence of an organic acid such as formic acid, acetic acid, propionic acid, butyric acid, maleic acid, fumaric acid, malonic acid, succinic acid, benzoic acid, meta-chlorobenzoic acid, p-nitrobenzoic acid and phthalic acid, at a temperature of from -70°C to the boiling point of the solvent used, generally from -50°C to room temperature, preferably from -20°C to 0°C for about 5 minutes to 24 hours, preferably about 5 minutes to 20 hours, or in water or an alcohol solvent such as ethanol, methanol and propanol, in the presence of an alkali such as alkali hydroxide (e.g. sodium hydroxide, potassium hydroxide and calcium hydroxide) at a temperature of from -70°C to the boiling point of the solvent used, generally from -50°C to room temperature, preferably from -20°C to 10°C for about 5 minutes to 24 hours, preferably about 1 hour to 10 hours.

Method 2: A compound of the formula (I-2)

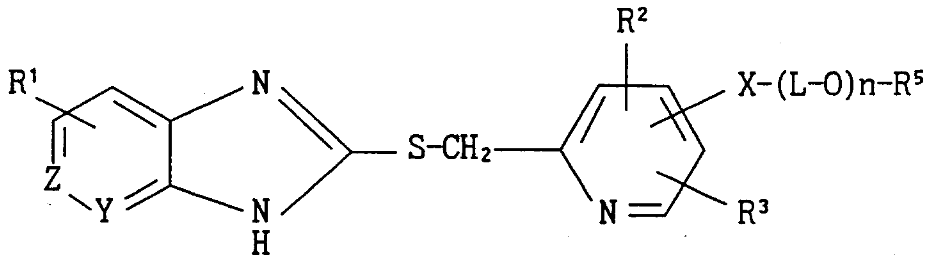

(I-2)

wherein each symbol is as defined above, is reacted with alkoxycarbonyl halide, haloalkyl alcohol or acyloxyhaloalkyl in the presence of an acid scavenger, or a compound of the formula (I-2) is reacted with formaldehyde to give a compound of the formula (I) wherein $R^4$ is alkoxycarbonyl, hydroxyalkyl (hydroxymethyl when reacted with formaldehyde) or acyloxyalkyl (m=0).

Examples of alkoxycarbonyl halide include halides corresponding to alkoxycarbonyl at $R^4$, such as ethoxycarbonyl chloride and isobutoxycarbonyl chloride.

Examples of haloalkyl alcohol include halides corresponding to hydroxyalkyl at $R^4$, such as 2-chloroethanol, 2-bromoethanol, 3-chloropropanol and 3-bromopropanol.

Examples of acyloxyhaloalkyl include halides corresponding to acyloxyalkyl at $R^4$, such as acetyloxymethyl bromide, 2-acetyloxyethyl bromide and 4-acetyloxybutyl iodide.

Examples of acid scavenger include sodium hydride, sodium hydroxide, potassium hydroxide, sodium methylate, potassium carbonate and metallic sodium.

This reaction is generally carried out in a solvent inert to the reaction such as N,N-dimethylformamide, ethanol, methanol, chloroform and water at -20°C-140°C, preferably 0°C-80°C, for about 10 minutes to 24 hours, preferably for 30 minutes to 6 hours.

The compound (I) thus produced can be separated and purified by a conventional method such as recrystallization and column chromatography.

The optical isomer of the compound (I) of the present invention can be produced by subjecting a reaction product to fractional crystallization and the like, or by subjecting a starting compound optically resolved in advance to the above-mentioned reaction.

The compound (I) of the present invention can be converted into the above-mentioned acid addition salt by treating with hydrochloric acid, hydrobromic acid, hydroiodic acid, sulfuric acid, nitric acid, phosphoric acid, acetic acid, citric acid, maleic acid, fumaric acid, malonic acid, malic acid, tartaric acid, succinic acid, methanesulfonic acid and the like by a conventional method. By reacting with sodium hydroxide, potassium hydroxide, calcium hydroxide, magnesium hydroxide and the like, the corresponding metallic salt can be obtained.

The compound of the formula (II) can be obtained, for example, by the following reaction scheme.

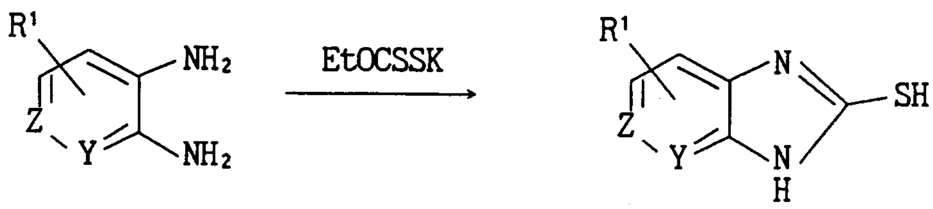


(VIII) (II')

wherein each symbol is as defined above.

That is, a compound of the formula (VIII) and potassium xanthate are reacted in a solvent inert to the reaction such as water, methanol and ethanol at from 0°C to the boiling point of the solvent used, preferably 30-80°C, for about 10 minutes to 24 hours, preferably 1-5 hours, to give a compound of the formula (II) wherein $R^4$ is hydrogen, namely, the compound of the formula (II').

The compound of the formula (III) can be obtained, for example, by the following reaction scheme.

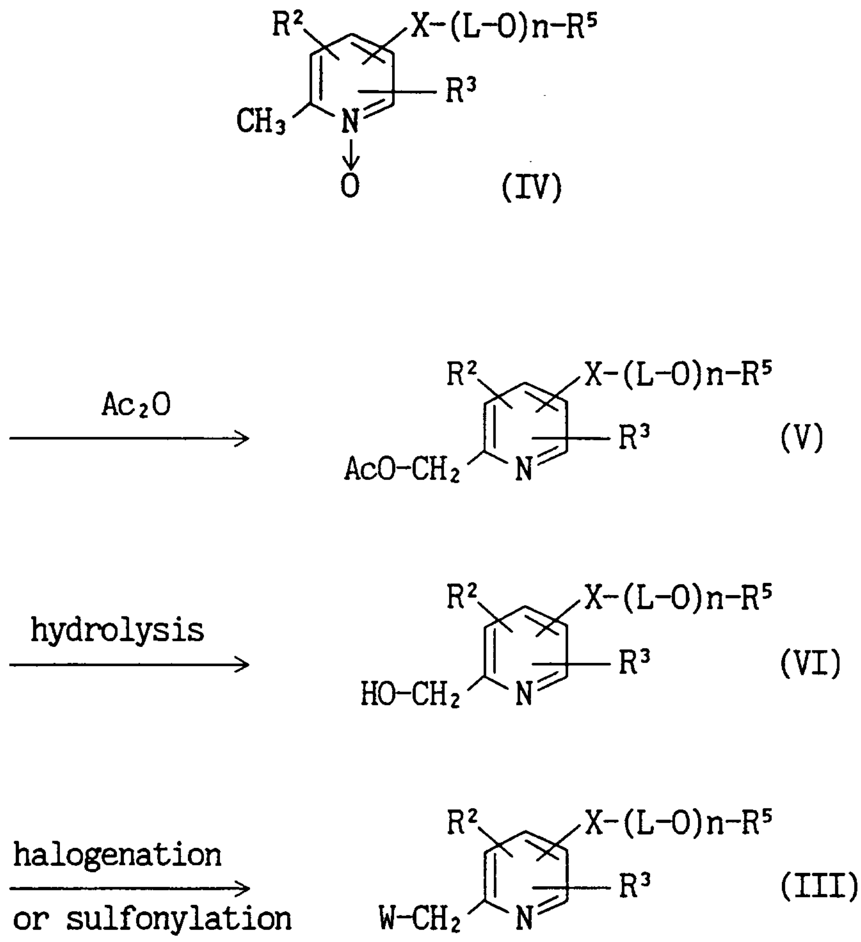


(IV) (V) (VI) (III)

wherein Ac is acetyl and other symbols are as defined above.

That is, a compound of the formula (IV) is heated with acetic anhydride to give a compound of the formula (V) which is hydrolyzed with sodium hydroxide and the like, and treated with a halogenizing agent such as thionyl chloride or a sulfonylating agent such as methanesulfonyl chloride to give a compound of the formula (III).

A compound of the formula (IV) wherein L is ethylene can be produced, for example, by the following reaction scheme.

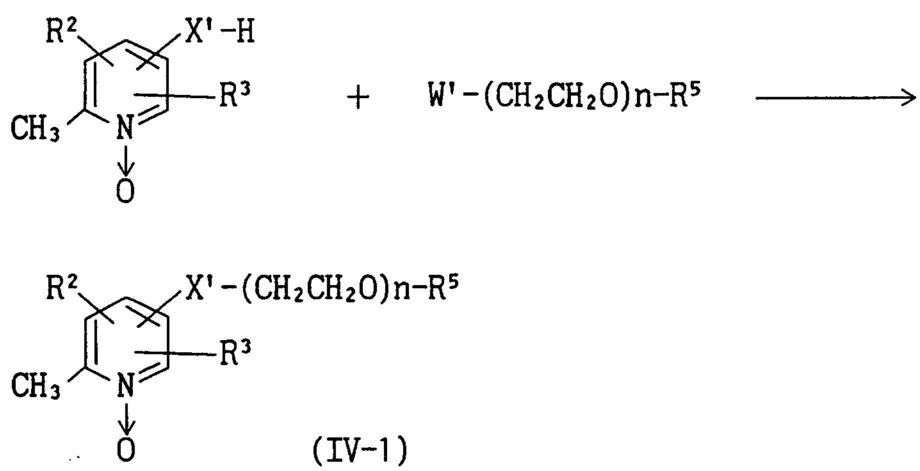

(IV-1)

wherein X' is sulfur atom, W' is a leaving group such as chlorine, bromine, iodine, methanesulfonyloxy and p-toluenesulfonyloxy, and other symbols are as defined above.

Alternatively,

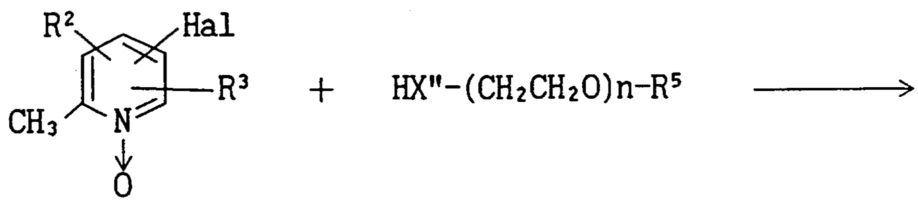

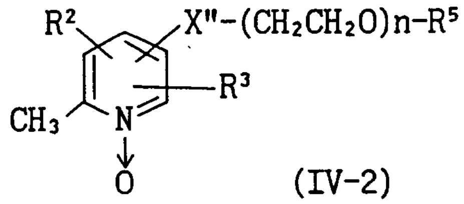

(IV-2)

wherein Hal is chlorine, bromine or iodine, X" is oxygen atom and other symbols are as defined above.

Every reaction mentioned above generally proceeds in a solvent inert to the reaction such as water, alcohols (e.g., methanol and ethanol), chloroform, dichloromethane, benzene, toluene, dimethylformamide and a mixed solvent thereof, in the presence of a base such as sodium hydroxide, potassium hydroxide, sodium hydride, sodium methoxide, sodium ethoxide, sodium carbonate, potassium carbonate, metallic sodium, triethylamine and pyridine, at from 0°C to the boiling point of the solvent used, preferably 20-80°C for about 10 minutes to 24 hours, preferably from 30 minutes to 7 hours.

The compound of the formula (VI) wherein X is SO or $SO_2$ can be produced by subjecting a compound of the formula (IV) wherein X is sulfur atom to oxidation.

Examples of the oxidizing agent to be used for the oxidation include meta-chloroperbenzoic acid, peracetic acid, trifluoroperacetic acid, permaleic acid, potassium hyperoxide, sodium bromite, sodium hypobromite and hydrogen peroxide.

Said oxidation reaction is generally carried out in a solvent inert to the reaction, such as water, dichloromethane, chloroform, tetrahydrofuran, dioxane, dimethylformamide and a mixed solvent thereof, in the presence of an organic acid such as formic acid, acetic acid, propionic acid, butyric acid, maleic acid, fumaric acid, malonic acid, succinic acid, benzoic acid, meta-chlorobenzoic acid, p-nitrobenzoic acid and phthalic acid, at a temperature of from -70°C to the boiling point of the solvent used, preferably from -50°C to room temperature, more preferably from -20°C to 0°C for about 5 minutes to 24 hours, preferably about 5 minutes to 20 hours, or in water or an alcohol solvent such as ethanol, methanol and propanol, in the presence of an alkali such as alkali hydroxide (e.g. sodium hydroxide, potassium hydroxide and calcium hydroxide) at a temperature of from -70°C to the boiling point of the solvent used, preferably from -50°C to room temperature, more preferably from -20°C to 10°C for about 5 minutes to 24 hours, preferably 1 hour to 10 hours.

A compound of the formula (IV) wherein L is vinylene can be produced, for example, by the following reaction scheme.

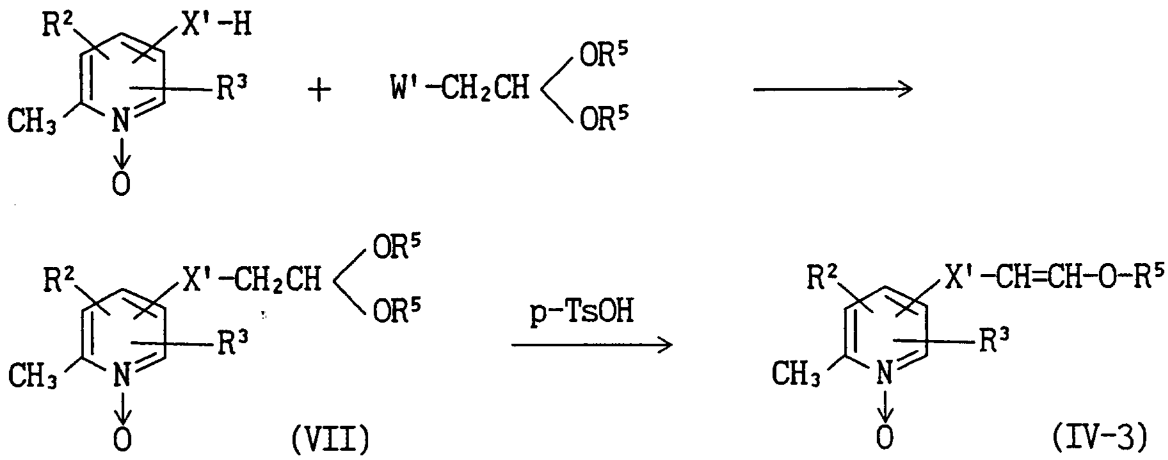


(VII) (IV-3)

wherein each symbol is as defined above.

Alternatively,

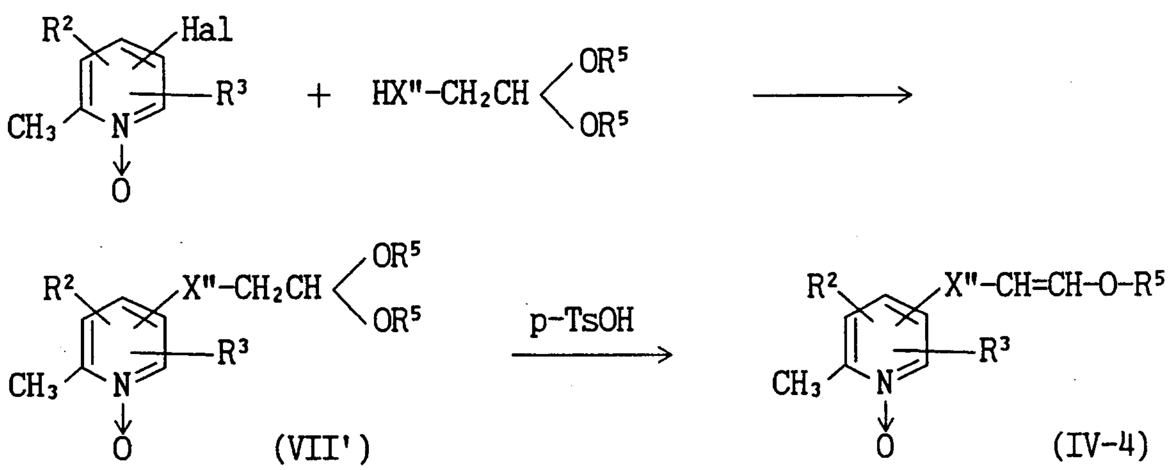


(VII') (IV-4)

wherein each symbol is as defined above.

In every reaction mentioned above, the first step reaction generally proceeds in a solvent inert to the reaction, such as water, alcohols (e.g., methanol and ethanol), chloroform, dichloromethane, benzene, toluene, dimethylformamide and a mixed solvent thereof, in the presence of a base such as sodium hydroxide, potassium hydroxide, sodium hydride, sodium methoxide, sodium ethoxide, sodium carbonate, potassium carbonate, metallic sodium, triethylamine and pyridine, at from about 0°C to the boiling point of the solvent used, preferably 20-80°C for about 10 minutes to 24 hours, preferably from 30 minutes to 7 hours. The compounds of the formulas (VII) and (VII') are reacted in a nonaqueous solvent such as benzene and toluene in the presence of an acid such as p-toluenesulfonic acid (p-TsOH) at from 0°C to the boiling point of the solvent used, preferably at the boiling point of the solvent used, for about 30 minutes to 48 hours, preferably from 3 hours to 12 hours, to give the compounds of the formulas (IV-3) and (IV-4).

The compound (I) and pharmaceutically acceptable salts thereof of the present invention show selective antibacterial activity against the bacteria belonging to the genus *Helicobacter* represented by *Helicobacter pylori*, and are useful for the prevention and treatment of various diseases caused by *Helicobacter*.

That is, the compound (I) and pharmaceutically acceptable salts thereof of the present invention are usable for mammals inclusive of human for the prevention and treatment of various diseases caused by the bacteria belonging to the genus *Helicobacter*, the prevention of recurrence and relapse of ulcer, suppression of vomiting, the prevention and treatment of non-ulcer dyspepsia, and the prevention and treatment of tumor (e.g., carcinoma of stomach) and the like.

In addition, the compound (I) and pharmaceutically acceptable salts thereof of the present invention have antiulcer activity, gastrointestinal cytoprotective activity, gastric acid secretion-suppressive activity and anti-diarrhea activity, and are useful for the prevention and treatment of gastrointestinal diseases such as gastric ulcer, duodenal ulcer, gastritis,

diarrhea and colitis. Moreover, they have low toxicity, are stable to acid etc. and show small increase in gastrin value in blood.

Such pharmacological actions of the compound (I) and pharmaceutically acceptable salts thereof of the present invention can be confirmed by the method of Ghosh et al, Br. J. Pharmacol. *13*, p 54 (1958).

When the compound (I) or a pharmaceutically acceptable salt thereof of the present invention is used as a pharmaceutical preparation, a therapeutically effective amount of compound (I) or a pharmaceutically acceptable salt thereof is admixed with pharmaceutically acceptable additives such as excipients, carriers, diluents and solubilizers and formulated into capsules, tablets (inclusive of sugar-coated tablets and film-coated tablets), granules, injections or infusions for administration. The dose is generally about 0.01-30 mg/kg, preferably 0.1-3 mg/kg daily for an adult by oral administration, though it is subject to change depending on the symptom, age, resistance to drug etc. of the patients.

The present invention is explained in detail by way of Experimental Examples and Examples in the following. It is needless to say that the present invention is not limited to them.

Experimental Example 1: Antibacterial activity (*in vitro*) against *Helicobacter pylori*

Clinical isolates (18 strains) were incubated using media supplemented with 5% horse serum under a microaerophilic environment at 37°C for 72 hours and diluted with Brucella broth to give bacterial cultures having a concentration of approximately $10^6$ cell/ml. The diluted bacterial culture was spot inoculated with a microplanter on an agar plate containing 2-fold dilution concentration series of the test compound. The cells were incubated at 37°C under 8% $CO_2$ for 3-4 days. The minimum inhibitory concentration (MIC) was determined, based on which $MIC_{50}$ (minimum concentration necessary for inhibiting growth by 50%) and $MIC_{90}$ (minimum concentration necessary for inhibiting growth by 90%) were calculated. The results are shown in Table 1.

Table 1

| Test compound (Example No.) | $MIC_{50}$ (μg/ml) | $MIC_{90}$ (μg/ml) |
|---|---|---|
| 17 | 0.025 | 0.05 |
| 21 | 0.012 | 0.025 |
| 22 | 0.025 | 0.025 |

Experimental Example 2: Action on gastric acid secretion of rats subjected to gastroperfusion

The experiment was done according to the method of Ghosh et al, Br. J. Pharmacol. *13*, p 54 (1958). Wistar rats were fasted for 20 hours and anesthetized with urethane (1.5 g/kg, subcutaneous). A polyethylene tube for injection and discharge for gastroperfusion was attached to the rats. Gastroperfusion was performed using physiological saline (37°C) at a speed of 7 ml/min and the perfusate was recovered at 10-min intervals. Histamine hydrochloride (1 mg/kg) was intravenously administered at 1-hr intervals to induce secretion of gastric acid. A test solution containing the test compound was intravenously administered 5 minutes before the histamine hydrochloride administration. The acidity of the gastric acid was titrated up to pH 7.0 with an automatic titrator and percent suppression of gastric acid secretion by the test compound was determined. The results are shown in Table 2.

Table 2

| Test compound (Example No.) | Dose (mg/kg i.v.) | Suppression (%) |
|---|---|---|
| 25 | 3 | 100 |
| 26 | 3 | 50 |

Example 1

60% Sodium hydride (1.65 g) was added to polyethylene glycol monomethyl ether (55 g, average molecular weight 350) with stirring under ice-cooling, and 4-chloro-2,3-dimethylpyridine-N-oxide (5.0 g) was added at room temperature, which was followed by stirring at 80-90°C for 2 hours. After the completion of the reaction, water was added and the mixture was extracted with ethyl acetate. The ethyl acetate layer was dried over anhydrous magnesium sulfate. The solvent was distilled away, and the residue was subjected to silica gel column chromatography and eluted with chloroform-methanol=95:5 to give 7.2 g of 2,3-dimethyl-4-(2-methoxypolyethoxy)pyridine-N-oxide (derived from polyethylene glycol monomethyl ether having an average molecular weight of 350) as a pale-yellow oil.

$^1$H-NMR($CDCl_3$):δ(ppm)=2.2(s,3H), 2.5(s,3H), 3.38(s,3H), 3.48-3.8(m), 3.9(t,2H), 4.15(t,2H), 6.65(d,1H), 8.1(d,1H)

In the same manner, the following compounds were obtained.

Example 2

2,3-dimethyl-4-(2-methoxypolyethoxy)pyridine-N-oxide (derived from polyethylene glycol monomethyl ether having an average molecular weight of 550)

$^1$H-NMR($CDCl_3$):δ(ppm)=2.2(s,3H), 2.5(s,3H), 3.38(s,3H), 3.4-3.75(m), 3.9(t,2H), 4.15(t,2H), 6.65(d,1H), 8.1(d,1H)

Example 3

2,3-dimethyl-4-(2-methoxypolyethoxy)pyridine-N-oxide (derived from polyethylene glycol monomethyl ether having an average molecular weight of 750)

$^1$H-NMR($CDCl_3$):δ(ppm)=2.2(s,3H), 2.5(s,3H), 3.38(s,3H), 3.5-3.7(m), 3.9(t,2H), 4.1(t,2H), 6.65(d,1H), 8.1(d,1H)

Example 4

Metallic sodium (3.86 g) was added to ethanol (300 ml) and 2,3-dimethyl-4-mercaptopyridine-N-oxide (23.7 g) was added with stirring at room temperature, which was followed by stirring at 50°C for 15 minutes. 2-Methoxypolyethoxy ethyl chloride (56.2 g) obtained by reacting polyethylene glycol monomethyl ether (an average molecular weight 350) and thionyl chloride by a conventional method was added to the aforementioned reaction mixture, and the mixture was refluxed under stirring for 2 hours. After the completion of the reaction, the reaction mixture was concentrated under reduced pressure. Water was added and the mixture was extracted with ethyl acetate. The ethyl acetate layer was dried over anhydrous magnesium sulfate and concentrated under reduced pressure to give 74 g of 2,3-dimethyl-4-(2-(2-methoxypolyethoxy)ethylthio)pyridine-N-oxide (derived from polyethylene glycol monomethyl ether having an average molecular weight of 350) as a pale-yellow oil.

$^1$H-NMR($CDCl_3$):δ(ppm)=2.35(s,3H), 2.52(s,3H), 3.17(t,2H), 3.38(s,3H), 3.45-3.8(m), 7.05(d,1H), 8.05(d,1H)

In the same manner, the following compounds were obtained.

Example 5

2,3-dimethyl-4-(2-(2-methoxyethoxy)ethylthio)pyridine-N-oxide

$^1$H-NMR($CDCl_3$):δ(ppm)=2.35(s,3H), 2.55(s,3H), 3.17(t,2H), 3.40(s,3H), 3.52-3.8(m,6H), 7.05(d,1H), 8.05(d,1H)

Example 6

2,3-dimethyl-4-(2-(2-(2-methoxyethoxy)ethoxy)ethylthio)pyridine-N-oxide

$^1$H-NMR($CDCl_3$):δ(ppm)=2.35(s,3H), 2.55(s,3H), 3.17(t,2H), 3.38(s,3H), 3.45-3.8(m,10H), 7.05(d,1H), 8.05(d,1H)

Example 7

2,3-dimethyl-4-(2-(2-methoxypolyethoxy)ethylthio)pyridine-N-oxide (derived from polyethylene glycol monomethyl ether having an average molecular weight of 550)

$^1$H-NMR($CDCl_3$):δ(ppm)=2.25(s,3H), 2.35(s,3H), 3.20 (t,2H), 3.35(s,3H), 3.45-3.6(m), 7.2(d,1H), 8.05(d,1H)

Example 8

2,3-dimethyl-4-(2-(2-methoxypolyethoxy)ethylthio)pyridine-N-oxide (derived from polyethylene glycol mOnomethyl ether having an average molecular weight of 750)

$^1$H-NMR($CDCl_3$):δ(ppm)=2.35(s,3H), 2.55(t,2H), 3.15(t,2H), 3.35(s,3H), 3.50-3.75(m), 7.05(d,1H), 8.10(d,1H)

Example 9

2,3-Dimethyl-4-(2-(2-methoxypolyethoxy)ethylthio)pyridine-N-oxide (7.2 g, derived from polyethylene glycol monomethyl ether having an average molecular weight of 350) was dissolved in acetic anhydride (30 ml) and the mixture was stirred at 90°C for 2 hours. The solvent was distilled away. Water was added to the residue, and the mixture was extracted with chloroform, dried over anhydrous magnesium sulfate and the solvent was distilled away. The obtained residue, 2-acetoxymethyl-3-methyl-4-(2-(2-methoxypolyethoxy)ethylthio)pyridine, was dissolved in ethanol (80 ml) and sodium hydroxide (0.9 g) dissolved in water (10 ml) was added, which was followed by refluxing under stirring for one hour. After the completion of the reaction, ethanol was distilled away and the residue was extracted with ethyl acetate. The ethyl acetate layer was dried over anhydrous magnesium sulfate and the solvent was distilled away. The residue was subjected to silica gel column chromatography and eluted with chloroform-methanol=98:2 to give 4.6 g of 3-methyl-4-(2-(2-methoxypolyethoxy)ethylthio)pyridine-2-methanol (derived from polyethylene glycol monomethyl ether having an average molecular weight of 350) as a pale-yellow oil.

$^1$H-NMR($CDCl_3$):δ(ppm)=2.15(s,3H), 3.20(t,2H), 3.39(s,3H), 3.45-3.85(m), 4.48(s,2H), 7.05(d,1H), 8.25(d,1H)

In the same manner, the following compounds were obtained.

Example 10

3-methyl-4-(2-methoxypolyethoxy)pyridine-2-methanol (derived from polyethylene glycol monomethyl ether having an average molecular weight of 550)

$^1$H-NMR($CDCl_3$):δ(ppm)=2.05(s,3H), 3.38(s,3H), 3.4-3.8(m), 3.9(t,2H), 4.15(t,2H), 4.63(s,2H), 6.7(d,1H), 8.25(d,1H)

Example 11

3-methyl-4-(2-methoxypolyethoxy)pyridine-2-methanol (derived from polyethylene glycol monomethyl ether having an average molecular weight of 750)

$^1$H-NMR($CDCl_3$):δ(ppm)=2.05(s,3H), 3.38(s,3H), 3.4-3.75(m), 3.9(t,2H), 4.15(t,2H), 4.62(s,2H), 6.7(d,1H), 8.25(d,1H)

Example 12

3-methyl-4-(2-(2-methoxyethoxy)ethylthio)pyridine-2-methanol

$^1$H-NMR($CDCl_3$):δ(ppm)=2.15(s,3H), 3.2(t,3H), 3.4(s,3H), 3.52(t,2H), 3.65(t,2H), 3.75(t,2H), 4.65(s,2H), 7.05(d,1H), 8.23(d,1H)

Example 13

3-methyl-4-(2-(2-(2-methoxyethoxy)ethoxy)ethylthio)pyridine-2-methanol

$^1$H-NMR($CDCl_3$):δ(ppm)=2.15(s,3H), 3.2(t,2H), 3.39(s,3H), 3.45-3.85(m,10H), 4.65(s,2H), 7.05(d,1H), 8.25(d,1H)

Example 14

3-methyl-4-(2-(2-methoxypolyethoxy)ethylthio)pyridine-2-methanol (derived from polyethylene glycol monomethyl ether having an average molecular weight of 350)

$^1$H-NMR($CDCl_3$):δ(ppm)=2.15(s,3H), 3.2(t,2H), 3.39(s,3H), 3.5-3.8(m), 4.68(s,2H), 7.1(d,1H), 8.25(d,1H)

Example 15

3-methyl-4-(2-(2-methoxypolyethoxy)ethylthio)pyridine-2-methanol (derived from polyethylene glycol monomethyl ether having an average molecular weight of 550)

$^1$H-NMR(d6-DMSO):δ(ppm)=2.2(s,3H), 3.25(t,2H), 3.3(s,3H), 3.4-3.8(m), 4.53(d,1H), 5.0(t,1H), 7.22(d,1H), 8.2(d,1H)

Example 16

3-methyl-4-(2-(2-methoxypolyethoxy)ethylthio)pyridine-2-methanol (derived from polyethylene glycol monomethyl ether having an average molecular weight of 750)

$^1$H-NMR(d6-DMSO):δ(ppm)=2.13(s,3H), 3.23(t,2H), 3.38(s,3H), 3.5-3.8(m), 3.70(s,2H), 7.08(d,1H), 8.25(d,1H)

Example 17

3-Methyl-4-(2-(2-methoxyethoxy)ethylthio)pyridine-2-methanol (10 g) was dissolved in chloroform (50 ml). Thionyl chloride (3.1 ml) was dropwise added under ice-cooling and the mixture was stirred at room temperature for 1.5 hours. After the completion of the reaction, thionyl chloride was distilled away. The residue was alkali-supersaturated with potassium carbonate and extracted with chloroform. The chloroform layer was dried over anhydrous magnesium sulfate and the solvent was distilled away to give 10 g of 2-chloromethyl-3-methyl-4-(2-(2-methoxypolyethoxy)ethylthio)pyridine as an oil. The oil was added to ethanol (50 ml) containing a solution of 2-mercaptobenzimidazole (2.5 g) and sodium hydroxide (0.8 g) dissolved in water (10 ml), and the mixture was refluxed under heating for 2 hours. After the completion of the reaction, ethanol was distilled away and water was added to the residue, followed by extraction with chloroform. The chloroform layer was dried over anhydrous magnesium sulfate and the solvent was distilled away. Ethanolic hydrochloric acid was added to the residue to give a hydrochloride, which was recrystallized from ethanol to give 2-((3-methyl-4-(2-(2-methoxyethoxy)ethylthio)-2-pyridyl)methylthio)-1H-benzimidazole • hydrochloride as white crystals.

Melting point 186-189°C

Example 18

3-Methyl-4-(2-methoxypolyethoxy)pyridine-2-methanol (2.3 g, derived from polyethylene glycol monomethyl ether having an average molecular weight of 350) was dissolved in chloroform (50 ml) and thionyl chloride (3 ml) was dropwise added under ice-cooling and the mixture was stirred at room temperature for 1.5 hours. After the completion of the reaction, thionyl chloride was distilled away and the residue was alkali-supersaturated with potassium carbonate and extracted with chloroform. The chloroform layer was dried over anhydrous magnesium sulfate and the solvent was distilled away to give 2-chloromethyl-3-methyl-4-(2-methoxypolyethoxy)pyridine which was added to ethanol (30 ml) containing a solution of 2-mercaptobenzimidazole (0.68 g) and sodium hydroxide (0.38 g) dissolved in water (10 ml), and the mixture was refluxed under heating for one hour. After the completion of the reaction, ethanol was distilled away and water was added to the residue, followed by extraction with chloroform. The chloroform layer was dried over anhydrous magnesium sulfate and the solvent was distilled away. The residue was subjected to silica gel column chromatography and eluted with chloroform-methanol=97:3 to give 2-((3-methyl-4-(2-methoxypolyethoxy)-2-pyridyl)methylthio)-1H-benzimidazole as a pale-yellow oil (derived from polyethylene glycol monomethyl ether having an average molecular weight of 350).

$^1$H-NMR($CDCl_3$):δ(ppm)=2.3(s,3H), 3.4(s,3H), 3.5-3.8(m), 3.9(t,2H), 4.2(t,2H), 4.4(s,2H), 6.75(d,1H), 7.15-7.25(m,2H), 7.5-7.6(m,2H), 8.35(d,1H)

In the same manner, the following compounds were obtained.

Example 19

2-((3-methyl-4-(2-methoxypolyethoxy)-2-pyridyl)methylthio)-1H-benzimidazole (derived from polyethylene glycol monomethyl ether having an average molecular weight of 550)

$^1$H-NMR($CDCl_3$):δ(ppm)=2.25(s,3H), 3.37(s,3H), 3.4-3.8(m), 3.9(t,2H), 4.2(t,2H), 4.4(s,2H), 6.75(d,1H), 7.1-7.3(m,2H), 7.4-7.6(m,2H), 8.3(d,1H)

Example 20

2-((3-methyl-4-(2-methoxypolyethoxy)-2-pyridyl)methylthio)-1H-benzimidazole (derived from polyethylene glycol monomethyl ether having an average molecular weight of 750)

$^1$H-NMR($CDCl_3$):δ(ppm)=2.3(s,3H), 3.4(s,3H), 3.45-3.75(m), 3.9(t,2H), 4.2(t,2H), 4.4(s,2H), 6.75(d,1H), 7.1-7.2(m,2H), 7.45-7.6(m,2H), 8.35(d,1H)

Example 21

2-((3-methyl-4-(2-(2-(2-methoxyethoxy)ethoxy)ethylthio)-2-pyridyl)methylthio)-1H-benzimidazole • hydrochloride
Melting point 181-183°C

Example 22

2-((3-methyl-4-(2-(2-methoxypolyethoxy)ethylthio)-2-pyridyl)methylthio)-1H-benzimidazole • hydrochloride (derived from polyethylene glycol monomethyl ether having an average molecular weight of 350)

$^1$H-NMR($CDCl_3$):δ(ppm)=2.38(s,3H), 3.39(s,3H), 3.5-3.8(m), 4.5(s,2H), 7.1-7.6(m,5H), 8.3(d,1H)

Example 23

2-((3-methyl-4-(2-(2-methoxypolyethoxy)ethylthio)-2-pyridyl)methylthio)-1H-benzimidazole • hydrochloride (derived from polyethylene glycol monomethyl ether having an average molecular weight of 550)

$^1$H-NMR($CDCl_3$):δ(ppm)=2.38(s,3H), 3.39(s,3H), 3.5-3.8(m), 4.5(s,2H), 7.1-7.6(m,5H), 8.3(d,1H)

Example 24

2-((3-methyl-4-(2-(2-methoxypolyethoxy)ethylthio)-2-pyridyl)methylthio)-1H-benzimidazole • hydrochloride (derived from polyethylene glycol monomethyl ether having an average molecular weight of 750)

$^1$H-NMR($CDCl_3$):δ(ppm)=2.38(s,3H), 3.20(t,2H), 3.38(s,3H), 3.5-3.85(m),4.48(s,2H), 7.08-7.20(m,3H), 7.48-7.58(m,2H), 8.28(d,1H)

Example 25

2-((3-Methyl-4-(2-(2-methoxypolyethoxy)ethylthio)-2-pyridyl)methylthio)-1H-benzimidazole (1.2 g, derived from polyethylene glycol monomethyl ether having an average molecular weight of 350) was dissolved in chloroform (20 ml) and 80% meta-chloroperbenzoic acid (0.38 g) was added under ice-cooling, which was followed by stirring under ice-cooling for 30 minutes. After the completion of the reaction, the solvent was distilled away. The residue was subjected to alumina column chromatography and eluted with chloroform-methanol=99:1 to give 0.52 g of 2-((3-methyl-4-(2-(2-methoxpolyethoxy)ethylthio)-2-pyridyl)methylsulfinyl)-1H-benzimidazole (derived from polyethylene glycol monomethyl ether having an average molecular weight of 350) as a pale-brown oil.

$^1$H-NMR($CDCl_3$):δ(ppm)=2.25(s,3H), 3.1-3.25(t,2H), 3.37(s,3H), 3.4-3.85(m), 4.8(d,2H), 7.05(d,1H), 7.20-7.40(m,4H), 8.25(d,1H)

Example 26

2-((3-Methyl-4-(2-methoxypolyethoxy)-2-pyridyl)methylthio)-1H-benzimidazole (0.34 g, derived from polyethylene glycol monomethyl ether having an average molecular weight of 350) was dissolved in chloroform (10 ml) and 80% meta-chloroperbenzoic acid (0.124 g) was added under ice-cooling, which was followed by stirring under ice-cooling for 30 minutes. After the completion of the reaction, the solvent was distilled away. The residue was subjected to alumina column chromatography and eluted with chloroform-methanol=97:3 to give 0.308 g of 2-((3-methyl-4-(2-methoxypolyethoxy)-2-pyridyl) methylsulfinyl)-1H-benzimidazole (derived from polyethylene glycol monomethyl ether having an average molecular weight of 350) as a pale-brown oil.

$^1$H-NMR($CDCl_3$):δ(ppm)=2.2(s,3H), 3.35(s,3H), 3.4-3.75(m), 3.85(t,2H), 4.1(t,2H), 4.75(d,2H), 6.7(d,1H), 7.20-7.35(m,4H), 8.25(d,1H)

In the same manner, the following compounds were obtained.

Example 27

2-((3-methyl-4-(2-methoxypolyethoxy)-2-pyridyl)methylsulfinyl)-1H-benzimidazole (derived from polyethylene glycol monomethyl ether having an average molecular weight of 550)

$^1$H-NMR($CDCl_3$):δ(ppm)=2.2(s,3H), 3.38(s,3H), 3.4-3.75(m), 3.85(t,2H), 4.15(t,2H), 4.75(d,2H), 6.7(d,1H), 7.20-7.40(m,4H), 8.15(d,1H)

Example 28

2-((3-methyl-4-(2-methoxypolyethoxy)-2-pyridyl)methylsulfinyl)-1H-benzimidazole (derived from polyethylene glycol monomethyl ether having an average molecular weight of 750)

$^1$H-NMR($CDCl_3$):δ(ppm)=2.2(s,3H), 3.38(s,3H), 3.4-3.75(m), 3.85(t,2H), 4.15(t,2H), 4.75(d,2H), 6.7(d,1H), 7.20-7.40(m,4H), 8.25(d,1H)

Example 29

2-((3-Methyl-4-(2-(2-methoxypolyethoxy)ethylthio)-2-pyridyl)methylthio)-1H-benzimidazole (1.2 g, derived from polyethylene glycol monomethyl ether having an average molecular weight of 550) was dissolved in chloroform (30 ml) and 80% meta-chloroperbenzoic acid (0.38 g) was added under ice-cooling, which was followed by stirring under ice-cooling for 30 minutes. After the completion of the reaction, the solvent was distilled away. The residue was subjected to alumina column chromatography and eluted with chloroform-methanol=99:1 to give 0.523 g of 2-((3-methyl-4-(2-(2-methoxypolyethoxy)ethylthio)-2-pyridyl)methylsulfinyl)-1H-benzimidazole (derived from polyethylene glycol monomethyl ether having an average molecular weight of 550) as a pale-brown oil.

$^1$H-NMR($CDCl_3$):δ(ppm)=2.25(s,3H), 3.15(t,2H), 3.37(s,3H), 3.4-3.75(m),3.73(t,2H), 4.78(d,2H), 7.06(d,1H), 7.20-7.40(m,4H), 8.25(d,1H)

Example 30

N,N-Dimethylformamide (200 ml), diethyl chloroacetal (25 g) and potassium carbonate (20 g) were added to 2,3-dimethyl-4-mercaptopyridine-N-oxide (20 g), and the mixture was stirred at 70-80°C for 12 hours. After the completion of the reaction, water was added and the mixture was extracted with ethyl acetate. The ethyl acetate layer was dried over anhydrous magnesium sulfate and the solvent was distilled away. The residue was subjected to silica gel column chromatography and eluted with chloroform-methanol=98:2 to give 28 g of 2,3-dimethyl-4-(2-diethoxyethylthio)pyridine-N-oxide as a pale-brown oil.

$^1$H-NMR($CDCl_3$):δ(ppm)=1.20(t,6H), 2.35(s,3H), 2.55(s,3H), 3.15(d,2H), 3.55(q,2H), 3.70(q,2H), 4.70(t,1H), 7.10(d,1H), 8.10(d,1H)

Example 31

2,3-Dimethyl-4-(2-diethoxyethylthio)pyridine-N-oxide (10.8 g) was dissolved in benzene (200 ml) and p-toluenesulfonic acid (7.6 g) was added, which was followed by refluxing under stirring for 2 hours. After the completion of the reaction, the reaction mixture was washed with water and dried over anhydrous magnesium sulfate. The solvent was distilled away, and the residu was subjected to silica gel column chromatography and eluted with chloroform-methanol=98:2 to give 2.1 g of trans-2,3-dimethyl-4-(2-ethoxyvinylthio)pyridine-N-oxide as a pale-yellow oil.

$^1$H-NMR($CDCl_3$):δ(ppm)=1.30(t,3H), 2.30(s,3H), 2.55(s,3H), 3.15(d,2H), 4.00(q,2H), 5.25(d,1H), 6.85(d,1H), 7.00(d,1H), 8.10(d,1H)

Example 32

In the same manner as in Example 9 using trans-2,3-dimethyl-4-(2-ethoxyvinylthio)pyridine-N-oxide, trans-3-methyl-4-(2-ethoxyvinylthio)pyridine-2-methanol was obtained.

$^1$H-NMR($CDCl_3$):δ(ppm)=1.35(t,3H), 2.15(s,3H), 4.00(q,2H), 4.65(s,2H), 5.30(d,1H), 6.85(d,1H), 7.10(d,1H), 8.25(d,1H)

In the same manner as in Example 17 or Example 18, the following compounds can be obtained.

Example 33

trans-2-((3-methyl-4-(2-ethoxyvinylthio)-2-pyridyl)methylthio)-1H-benzimidazole

Melting point 185-188°C (decomposition)

Example 34

2-((3-methyl-4-(2-(2-methoxypolyethoxy)ethylthio)-2-pyridyl)methylthio)-3H-imidazo[4,5-b]pyridine (derived from polyethylene glycol monomethyl ether having an average molecular weight of 350)

$^1$H-NMR($CDCl_3$):δ(ppm)=2.40(s,3H), 3.20(t,2H), 3.40(s,3H), 3.50-3.70(m), 3.75(t,2H), 4.50(s,2H), 7.15(m,2H), 7.85(d,1H), 8.30(d,1H), 8.35(d,1H)

Example 35

2-((3-methyl-4-(2-(2-methoxypolyethoxy)ethylthio)-2-pyridyl)methylthio)-3H-imidazo[4,5-c]pyridine (derived from polyethylene glycol monomethyl ether having an average molecular weight of 350)

$^1$H-NMR($CDCl_3$):δ(ppm)=2.40(s,3H), 3.25(t,2H), 3.40(s,3H), 3.50-3.70(m), 3.75(t,2H), 4.50(s,2H), 7.15(d,1H), 7.50(d,1H), 8.45(d,1H), 8.48(d,1H), 8.90(s,1H)

Example 36

2,3-dimethyl-4-(2-ethoxyethylthio)pyridine-N-oxide

$^1$H-NMR($CDCl_3$):δ(ppm)=1.20(t,3H), 2.35(s,3H), 2.56(s,3H), 3.15(t,2H), 3.52(q,2H), 3.68(t,2H), 7.04(d,1H), 8.10(d,1H)

Example 37

3-methyl-4-(2-ethoxyethylthio)pyridine-2-methanol

$^1$H-NMR($CDCl_3$):δ(ppm)=1.20(t,3H), 2.12(s,3H), 3.20(t,2H), 3.56(q,2H), 3.72(t,2H), 4.66(s,2H), 7.06(d,1H), 8.28(d,1H)

Example 38

2-((3-methyl-4-(2-ethoxyethylthio)-2-pyridyl)methylthio)-1H-benzimidazole • hydrochloride

Melting point 191-194°C (decomposition)

Example 39

2,3-dimethyl-4-(2-(2-(2-(2-methoxyethoxy)ethoxy)ethoxy)ethylthio)pyridine-N-oxide

$^1$H-NMR($CDCl_3$):δ(ppm)=2.35(s,3H), 2.55(s,3H), 3.17(t,2H), 3.40(s,3H), 3.50-3.80(m,14H), 7.05(d,1H), 8.05(d,1H)

Example 40

3-methyl-4-(2-(2-(2-(2-methoxyethoxy)ethoxy)ethoxy)ethylthio)pyridine-2-methanol

$^1$H-NMR($CDCl_3$):δ(ppm)=2.15(s,3H), 3.20(t,2H), 3.40(s,3H), 3.40-3.80(m,14H), 4.65(s,2H), 7.05(d,1H), 8.25(d,1H)

Example 41

2-((3-methyl-4-(2-(2-(2-(2-methoxyethoxy)ethoxy)ethoxy)ethylthio)-2-pyridyl)methylthio)-1H-benzimidazole • hydrochloride

$^1$H-NMR($CD_3OD$):δ(ppm)=2.37(s,3H), 3.22(s,3H), 3.30-3.90(m,16H), 5.15(s,2H), 7.30-7.50(m,2H), 7.55-7.75(m,3H), 8.45(d,1H)

The compounds obtained in the above Examples are shown in Tables 3 and 4, in which Me means methyl, Et means ethyl, Pr means propyl, Bu means butyl and ca. means *circa* (about).

Table 3

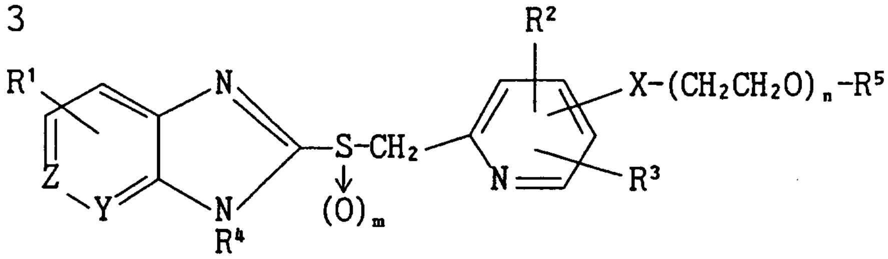

| Example | Z | Y | $R^1$ | $R^2$ | $R^3$ | $R^4$ | $R^5$ | X | m | n |
|---|---|---|---|---|---|---|---|---|---|---|
| 17 | CH | CH | H | 3-Me | H | H | Me | 4-S | 0 | 2 |
| 18 | CH | CH | H | 3-Me | H | H | Me | 4-O | 0 | ca.7 |
| 19 | CH | CH | H | 3-Me | H | H | Me | 4-O | 0 | ca.12 |
| 20 | CH | CH | H | 3-Me | H | H | Me | 4-O | 0 | ca.16 |
| 21 | CH | CH | H | 3-Me | H | H | Me | 4-S | 0 | 3 |
| 22 | CH | CH | H | 3-Me | H | H | Me | 4-S | 0 | ca.7 |
| 23 | CH | CH | H | 3-Me | H | H | Me | 4-S | 0 | ca.12 |
| 24 | CH | CH | H | 3-Me | H | H | Me | 4-S | 0 | ca.16 |
| 25 | CH | CH | H | 3-Me | H | H | Me | 4-S | 1 | ca.7 |
| 26 | CH | CH | H | 3-Me | H | H | Me | 4-O | 1 | ca.7 |
| 27 | CH | CH | H | 3-Me | H | H | Me | 4-O | 1 | ca.12 |
| 28 | CH | CH | H | 3-Me | H | H | Me | 4-O | 1 | ca.16 |
| 29 | CH | CH | H | 3-Me | H | H | Me | 4-S | 1 | ca.12 |
| 34 | CH | N | H | 3-Me | H | H | Me | 4-S | 0 | ca.7 |
| 35 | N | CH | H | 3-Me | H | H | Me | 4-S | 0 | ca.7 |
| 38 | CH | CH | H | 3-Me | H | H | Et | 4-S | 0 | 1 |
| 41 | CH | CH | H | 3-Me | H | H | Me | 4-S | 0 | 4 |

Table 4

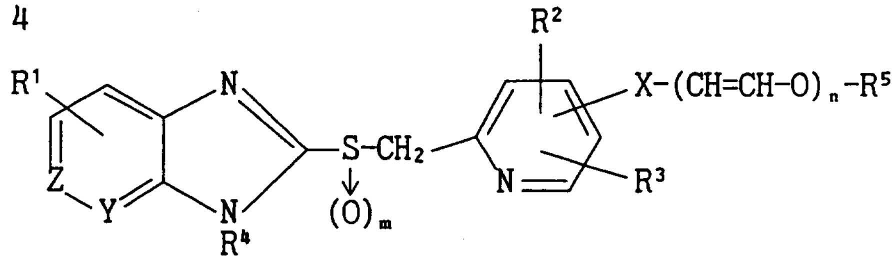

| Example | Z | Y | $R^1$ | $R^2$ | $R^3$ | $R^4$ | $R^5$ | X | m | n |
|---|---|---|---|---|---|---|---|---|---|---|
| 33 | CH | CH | H | 3-Me | H | H | Et | 4-S | 0 | 1 |

In the same manner as in the above Examples, the compounds of Tables 5 to 8 can be obtained.

Table 5

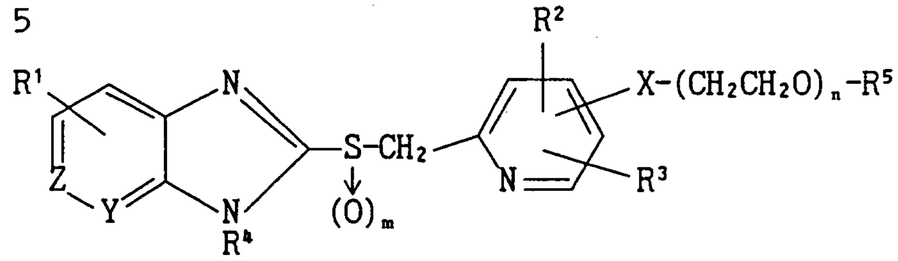

| No. | Z | Y | $R^1$ | $R^2$ | $R^3$ | $R^4$ | $R^5$ | X | m | n |
|---|---|---|---|---|---|---|---|---|---|---|
| 101 | N | CH | H | 3-Me | H | H | Me | 4-O | 0 | ca.7 |
| 102 | CH | N | H | 3-Me | H | H | Me | 4-O | 0 | ca.7 |
| 103 | CH | CH | H | 3-Me | H | $CH_2OH$ | Me | 4-S | 0 | ca.12 |
| 104 | CH | CH | H | 3-Me | H | $CH_2OH$ | Me | 4-O | 0 | ca.7 |
| 105 | CH | CH | H | 3-Me | H | H | Me | 4-SO | 0 | ca.7 |
| 106 | CH | CH | H | 3-Me | H | H | Me | 4-SO | 0 | ca.12 |
| 107 | CH | CH | H | 3-Me | H | H | Me | 4-$SO_2$ | 0 | ca.7 |
| 108 | CH | CH | H | 3-Me | H | H | Me | 4-$SO_2$ | 0 | ca.12 |
| 109 | CH | CH | H | 3-Me | H | H | Me | 4-O | 1 | ca.12 |
| 110 | CH | CH | H | 3-Me | H | H | Me | 4-O | 1 | ca.16 |
| 111 | CH | CH | H | 3-Me | H | H | Me | 4-O | 0 | 4 |
| 112 | CH | CH | H | 3-Me | H | H | Me | 4-O | 0 | 5 |
| 113 | CH | CH | H | 3-Me | H | H | Me | 4-O | 0 | 6 |
| 114 | CH | CH | H | 3-Me | H | H | Me | 4-O | 0 | 8 |
| 115 | CH | CH | H | 3-Me | H | H | Me | 4-S | 2 | 4 |

Table 6

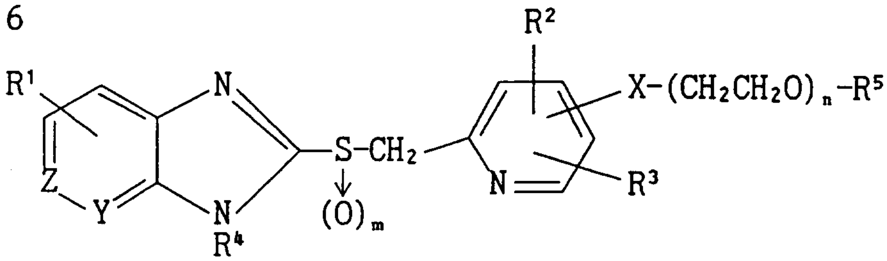

| No. | Z | Y | $R^1$ | $R^2$ | $R^3$ | $R^4$ | $R^5$ | X | m | n |
|---|---|---|---|---|---|---|---|---|---|---|
| 116 | CH | CH | H | 3-Me | H | H | Me | 4-S | 0 | 5 |
| 117 | CH | CH | H | 3-Me | H | H | Me | 4-S | 0 | 6 |
| 118 | CH | CH | H | 3-Me | H | H | Me | 4-S | 0 | 8 |
| 119 | CH | CH | H | H | H | H | Me | 4-O | 0 | ca.7 |
| 120 | CH | CH | H | H | H | H | Me | 4-S | 0 | ca.7 |
| 121 | CH | CH | H | H | H | H | Me | 4-O | 1 | ca.7 |
| 122 | CH | CH | H | H | H | H | Me | 4-S | 1 | ca.7 |
| 123 | CH | CH | H | 3-Me | H | H | Me | 4-O | 1 | 4 |
| 124 | CH | CH | H | 3-Me | H | H | Me | 4-O | 1 | 5 |
| 125 | CH | CH | H | 3-Me | H | H | Me | 4-O | 1 | 6 |
| 126 | CH | CH | H | 3-Me | H | H | Me | 4-O | 1 | 8 |
| 127 | CH | CH | H | 3-Me | H | H | Me | 4-S | 1 | 4 |
| 128 | CH | CH | H | 3-Me | H | H | Me | 4-S | 1 | 5 |
| 129 | CH | CH | H | 3-Me | H | H | Me | 4-S | 1 | 6 |
| 130 | CH | CH | H | 3-Me | H | H | Me | 4-S | 1 | 8 |

Table 7

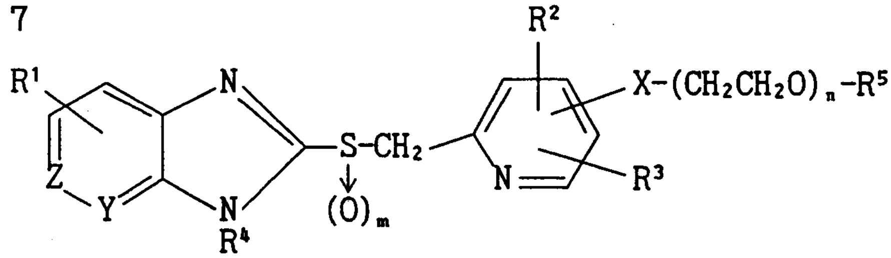

| No. | Z | Y | $R^1$ | $R^2$ | $R^3$ | $R^4$ | $R^5$ | X | m | n |
|---|---|---|---|---|---|---|---|---|---|---|
| 131 | CH | CH | H | 3-Me | 5-Me | H | Me | 4-O | 0 | ca.7 |
| 132 | CH | CH | H | 3-Me | 5-Me | H | Me | 4-S | 0 | ca.7 |
| 133 | CH | CH | H | 3-Me | 5-Me | H | Me | 4-O | 0 | ca.7 |
| 134 | CH | CH | H | 3-Me | 5-Me | H | Me | 4-S | 0 | ca.7 |
| 135 | CH | CH | 5-OMe | 3-Me | H | H | Me | 4-O | 0 | ca.7 |
| 136 | CH | CH | 5-OMe | 3-Me | H | H | Me | 4-S | 0 | ca.7 |
| 137 | CH | CH | 5-OH | 3-Me | H | H | Me | 4-O | 0 | ca.7 |
| 138 | CH | CH | 5-OH | 3-Me | H | H | Me | 4-S | 0 | ca.7 |
| 139 | CH | CH | H | 3-Me | 5-Me | H | H | 4-O | 0 | ca.7 |
| 140 | CH | CH | H | 3-Me | 5-Me | H | H | 4-S | 0 | ca.7 |
| 141 | CH | CH | H | 3-Me | 5-Me | COOEt | Me | 4-O | 0 | ca.7 |
| 142 | CH | CH | H | 3-Me | 5-Me | COOEt | Me | 4-S | 0 | ca.7 |
| 143 | CH | CH | 5-F | 3-Me | H | H | Me | 4-S | 0 | 3 |
| 144 | CH | CH | H | 3-Me | H | H | Me | 4-S | 1 | 2 |
| 145 | CH | CH | H | 3-Me | H | H | Me | 4-S | 1 | 3 |
| 146 | CH | CH | H | 3-Me | H | H | Me | 4-S | 0 | 1 |

Table 8

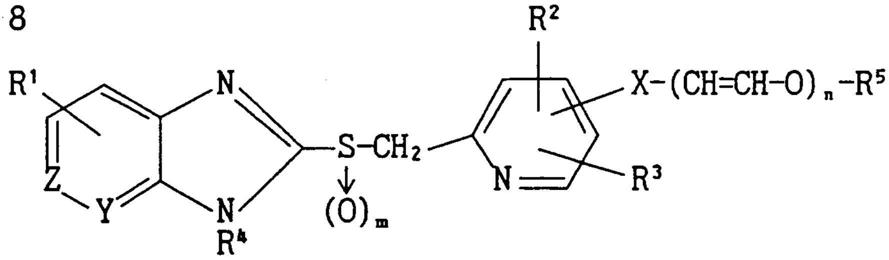

| No. | Z | Y | $R^1$ | $R^2$ | $R^3$ | $R^4$ | $R^5$ | X | m | n |
|---|---|---|---|---|---|---|---|---|---|---|
| 201 | CH | CH | H | 3-Me | H | H | Et | 4-O | 0 | 1 |
| 202 | CH | CH | H | 3-Me | H | H | Me | 4-S | 0 | 1 |
| 203 | CH | CH | H | 3-Me | H | H | Me | 4-S | 0 | 1 |
| 204 | CH | CH | H | 3-Me | H | H | Pr | 4-O | 0 | 1 |
| 205 | CH | CH | H | 3-Me | H | H | Pr | 4-S | 0 | 1 |
| 206 | CH | CH | H | 3-Me | H | H | Bu | 4-O | 0 | 1 |
| 207 | CH | CH | H | 3-Me | H | H | Bu | 4-S | 0 | 1 |
| 208 | CH | CH | H | H | H | H | Et | 4-S | 0 | 1 |
| 209 | CH | CH | H | 3-Me | 5-Me | H | Et | 4-S | 0 | 1 |
| 210 | CH | N | H | 3-Me | H | H | Et | 4-S | 0 | 1 |
| 211 | CH | CH | H | 3-Me | H | H | Et | 4-S | 1 | 1 |

Formulation Examples

Tablets The tablets containing 20 mg of the active ingredient are prepared according to the following formulation.

| | |
|---|---|
| Compound (I) | 20 mg |
| Corn starch | 15 mg |
| Lactose | 57 mg |
| Microcrystalline cellulose | 25 mg |
| Magnesium stearate | 3 mg |
| | 120 mg |

Capsules The capsules containing 20 mg of the active ingredient are prepared according to the following formulation.

| | |
|---|---|
| Compound (I) | 20 mg |
| Corn starch | 30 mg |
| Lactose | 63 mg |
| Hydroxypropylcellulose | 6 mg |
| Magnesium stearate | 1 mg |
| | 120 mg |

While the present invention has been adequately and fully explained by the foregoing specification and examples included therein, it is subject to changes and modifications as long as they fall within the spirit and scope of the present invention.

**Claims**

**1.** A pyridine compound of the formula (I)

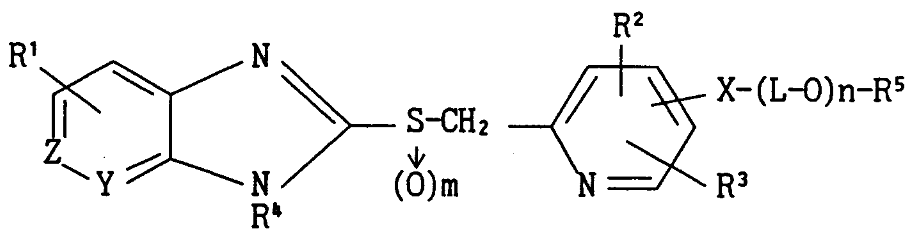
(I)

wherein

| | |
|---|---|
| $R^1$ | is a hydrogen, a halogen, an alkyl, an alkoxy, a hydroxyl, an alkoxycarbonyl, a carboxyl or a haloalkyl; |
| $R^2$ and $R^3$ | are the same or different and each is a hydrogen, a halogen or an alkyl; |
| $R^4$ | is a hydrogen, an alkoxycarbonyl, a hydroxyalkyl or an acyloxyalkyl; |
| $R^5$ | is a hydrogen or an alkyl; |
| X | is an oxygen atom, a sulfur atom, SO or $SO_2$; |
| Y and Z | are the same or different and each is CH or N; |
| L | is ethylene or vinylene; |
| m | is 0, 1 or 2; and |
| n | is an integer of from 1 to 1,000, |

provided that when L is vinylene, n is 1, and when $R^4$ is a hydrogen, X is an oxygen atom and L is ethylene, n is an integer of from 4 to 1,000, or a pharmaceutically acceptable salt thereof.

**2.** The pyridine compound of Claim 1, wherein the formula (I) is the formula (Ia)

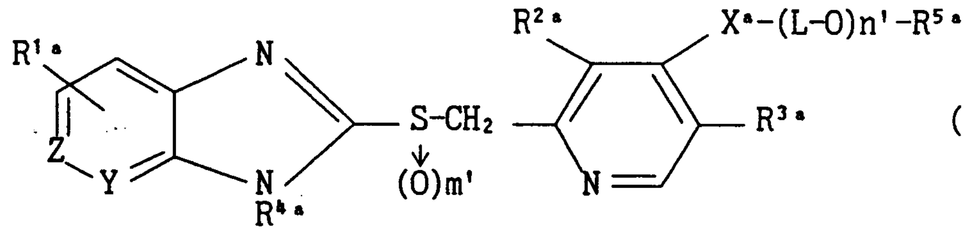
(Ia)

wherein

| | |
|---|---|
| $R^{1a}$ | is a hydrogen, a halogen, an alkyl having 1 to 5 carbon atoms, an alkoxy having 1 to 5 carbon atoms or a hydroxyl; |
| $R^{2a}$ and $R^{3a}$ | are the same or different and each is a hydrogen or an alkyl having 1 to 5 carbon atoms; |
| $R^{4a}$ | is a hydrogen; |
| $R^{5a}$ | is a hydrogen or an alkyl having 1 to 8 carbon atoms; |
| $X^a$ | is an oxygen atom or a sulfur atom; |
| Y and Z | are the same or different and each is CH or N; |
| L | is ethylene or vinylene; |
| m' | is 0 or 1; and |
| n' | is an integer of from 1 to 100, |

provided that when L is vinylene, n' is 1, and when $X^a$ is an oxygen atom and L is ethylene, n' is an integer of from 4 to 100, or a pharmaceutically acceptable salt thereof.

3. The pyridine compound of Claim 1, wherein the formula (I) is the formula (Ib)

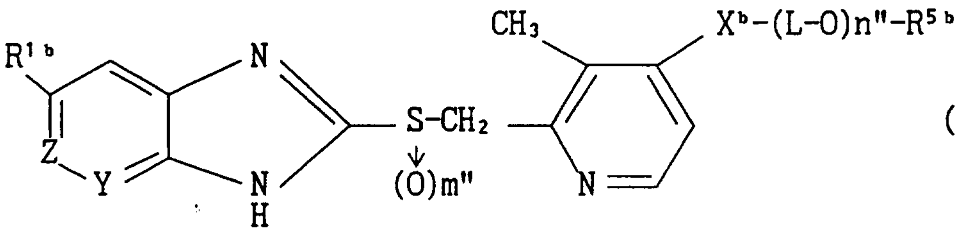

(Ib)

wherein

| | |
|---|---|
| $R^{1b}$ | is a hydrogen, a halogen, an alkoxy having 1 to 3 carbon atoms or hydroxyl; |
| $R^{5b}$ | is an alkyl having 1 to 5 carbon atoms; |
| $X^b$ | is an oxygen atom or a sulfur atom; |
| Y and Z | are the same or different and each is CH or N; |
| L | is ethylene or vinylene; |
| m'' | is 0 or 1; and |
| n'' | is an integer of from 1 to 20, |

provided that when L is vinylene, n'' is 1, and when $X^b$ is an oxygen atom and L is ethylene, n'' is an integer of from 4 to 20, or a pharmaceutically acceptable salt thereof.

4. The pyridine compound of Claim 3, wherein $X^b$ in the formula (Ib) is a sulfur atom, or a pharmaceutically acceptable salt thereof.

5. The pyridine compound of Claim 1, which is a member selected from the group consisting of

2-((3-methyl-4-(2-(2-methoxyethoxy)ethylthio)-2-pyridyl)methylthio)-1H-benzimidazole,
2-((3-methyl-4-(2-methoxypolyethoxy)-2-pyridyl)methylthio)-1H-benzimidazole derived from polyethylene glycol monomethyl ether having an average molecular weight of 350,
2-((3-methyl-4-(2-(2-(2-methoxyethoxy)ethoxy)ethylthio)-2-pyridyl)methylthio)-1H-benzimidazole,
2-((3-methyl-4-(2-(2-methoxypolyethoxy)ethylthio)-2-pyridyl)methylthio)-1H-benzimidazole derived from polyethylene glycol monomethyl ether having an average molecular weight of 350,
2-((3-methyl-4-(2-(2-methoxypolyethoxy)ethylthio)-2-pyridyl)methylthio)-1H-benzimidazole derived from polyethylene glycol monomethyl ether having an average molecular weight of 550,
2-((3-methyl-4-(2-(2-methoxypolyethoxy)ethylthio)-2-pyridyl)methylsulfinyl)-1H-benzimidazole derived from polyethylene glycol monomethyl ether having an average molecular weight of 350,
2-((3-methyl-4-(2-methoxppolyethoxy)-2-pyridyl)methylsulfinyl)-1H-benzimidazole derived from polyethylene glycol monomethyl ether having an average molecular weight of 350,
trans-2-((3-methyl-4-(2-ethoxyvinylthio)-2-pyridyl)methylthio)-1H-benzimidazole and

2-((3-methyl-4-(2-(2-methoxypolyethoxy)ethylthio)-2-pyridyl)methylthio)-3H-imidazo[4,5-b]pyridine derived from polyethylene glycol monomethyl ether having an average molecular weight of 350, or a pharmaceutically acceptable salt thereof.

**6.** A pharmaceutical composition comprising the pyridine compound of Claim 1 or a pharmaceutically acceptable salt thereof, and a pharmaceutically acceptable additive.

**7.** The pharmaceutical composition of Claim 6, which is an agent for the prophylaxis and treatment of various diseases caused by the bacteria belonging to the genus *Helicobacter*.

**8.** The pharmaceutical composition of Claim 6, which is an agent for the prophylaxis and treatment of gastrointestinal diseases.

## INTERNATIONAL SEARCH REPORT

International application No.
PCT/JP94/01800

**A. CLASSIFICATION OF SUBJECT MATTER**

Int. $Cl^6$ C07D401/12,471/04,A61K31/44

According to International Patent Classification (IPC) or to both national classification and IPC

**B. FIELDS SEARCHED**

Minimum documentation searched (classification system followed by classification symbols)

Int. $Cl^6$ C07D401/12, 471/04,A61K31/33

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | JP, A, 5-247035 (Eisai Co., Ltd.), September 24, 1993 (24. 09. 93), Claim &EP, A, 268956 | 1-8 |
| X | JP, A, 64-79177 (Eisai Co., Ltd.), March 24, 1989 (24. 03. 89), Claim &EP, A, 295603 | 1-8 |
| X | JP, A, 64-6270 (Eisai Co., Ltd.), January 10, 1989 (10. 01. 89), Claim &EP, A, 268956 | 1-8 |
| X | JP, A, 58-39622 (Aktiebolaget Hassle), March 8, 1983 (08. 03. 83), Claim &EP, A, 74341 | 1-8 |
| X | JP, A, 54-141783 (Aktiebolaget Hassle), November 5, 1979 (05. 11. 79), Claim &EP, A, 5129 | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 24, 1995 (24. 01. 95) | February 21, 1995 (21. 02. 95) |

| Name and mailing address of the ISA/ | Authorized officer |
|---|---|
| Japanese Patent Office | |
| Facsimile No. | Telephone No. |

Form PCT/ISA/210 (second sheet) (July 1992)